# EUROPÄISCHE PATENTSCHRIFT

(11) **EP 1 555 017 B1**
(45) Veröffentlichungstag und Bekanntmachung des Hinweises auf die Patenterteilung: **02.12.2009**
(21) Anmeldenummer: 04106547.5
(22) Anmeldetag: 14.12.2004
(51) Int. Cl.: A61K 8/06, A61K 8/86, A61Q 17/04

(54) **Dünnflüssige W/O Emulsionen**
Low-viscosity W/O Emulsions
Emulsions eau-dans-huile à faible viscosité

(30) Priorität: 19.01.2004 DE 102004002996
(43) Veröffentlichungstag der Anmeldung: 20.07.2005
(73) Patentinhaber: Beiersdorf AG, 20253 Hamburg (DE)
(72) Erfinder: Kröpke, Rainer, 22869, Schenefeld (DE); Bleckmann, Andreas, 22926, Ahrensburg (DE); Sugár, Martin, 20257 Hamburg (DE); Heptner, Astrid, 20257 Hamburg (DE); Tesch, Mirko, 22305 Hamburg (DE); Weingarz, Yvonne, 20251 Hamburg (DE)

(56) Entgegenhaltungen:
- WO-A-00/67888
- WO-A-03/070200
- DE-A1- 10 300 782
- US-A- 6 051 211

## Beschreibung

Die vorliegende Erfindung betrifft dünnflüssige, insbesondere sprühbare W/O Emulsionen.
In einer besonderen Ausführungsform betrifft die vorliegende Erfindung auch die Anwendung als Sonnenschutzprodukt.

Emulsionen gehören zu den dispersen Systemen. Emulsionen sind Zwei- oder Mehrphasensysteme von zwei oder mehr ineinander nicht oder nur wenig löslichen Flüssigkeiten. Die Flüssigkeiten (rein oder als Lösungen) liegen in einer Emulsion in einer mehr oder weniger feinen Verteilung vor, die im allgemeinen nur begrenzt stabil ist.

Bekannte dünnflüssige W/O-Emulsionen, d.h. mit einer Viskosität kleiner 2000 mPa s, bestimmbar mit einem Haake Viskotester VT-02 bei 25 °C, die insbesondere sprühbar sind, enthalten mindestens zwei Emulgatoren, die zur Stabilisierung der Emulsion miteinander kombiniert werden. Beispielsweise sind in DE 10154547 ein W/S-, ein W/O- und ein O/W-Emulgator zu kombinieren, um eine dünnflüssige Emulsion bereit stellen zu können.

Nach dem heutigen Stand der Technik ist es nicht möglich, stabile, sprühbare W/O-Emulsionen mit nur einem Emulgator herzustellen. Darüber hinaus weisen verfügbare sprühbare Emulsionen mit zwei oder mehreren Emulgatoren einige unerwünschte Nebeneffekt auf, wie beispielsweise eine erhöhte Formelinstabilität bei hohen als auch bei tiefen Temperaturen.

Aufgabe der vorliegenden Erfindung ist es daher eine dünnflüssige W/O-Emulsion bereit zu stellen, die eine verbesserte Lagerstabilität, insbesondere bei tieferen Temperaturen, aufweist.

Derzeitige Formulierungen enthalten zudem einen sehr hohen Gehalt an niedrig siedenden, sehr dünnflüssigen Silikonölen (z.B. 25-40 Gew.% Cyclomethicone) zur Gewährleistung der Sprühbarkeit, wie beispielsweise US 4,563,346 beschreibt. Diese Silikonöle bilden dann - fast ausschließlich - die kontinuierliche Phase der Emulsion.

Des weiteren werden zur Stabilisierung dünnflüssiger W/O-Emulsionen Schichtsilikate, z.B. WO 9614051, oder Organopolysiloxane, z.B. WO 9618374, eingesetzt. Dadurch ist es mit herkömmlichen Pumpsystemen nicht möglich, ein feines und gleichmäßiges Sprühbild zu erzeugen.

Ein weiterer Nachteil des Standes der Technik ist es, dass sich in derartigen Emulsionen, insbesondere mit hohem Silikonölgehalt, bei Raumtemperatur feste UV-Filter, wie beispielsweise aus der Gruppe der Triazine, nur sehr begrenzt lösen und sich daher nur Sonnenschutzprodukte mit geringem Lichtschutzfaktor (LSF, SPF) herstellen lassen.

Weiterer Nachteil ist, dass sehr dünnflüssige Emulsionen in der Regel nicht langzeitstabil sind und in Kombination mit einem hohen Anteil an Lichtschutzfiltern nach relativ kurzer Zeit, ca. 3 bis 6 Monate, eine irreversible Ölabscheidung auftritt, die bei höheren Lagertemperaturen sogar noch beschleunigt wird.

Die Einsatzkonzentration bekannter Lichtschutzfiltersubstanzen, die insbesondere auch im UV-A-Bereich eine hohe Filterwirkung zeigen, ist daher häufig - gerade in Kombination mit anderen zu lösenden Substanzen - be grenzt. Um z. B. hohe Mengen an öllöslichen UV-Filtersubstanzen einsetzen zu können, bräuchte man eine sehr große Ölphase (> 30 Gew.% bzw. über 40 Gew.%). Allerdings kann die hydrophobe Phase einer Emulsion - bei spielsweise einer W/O-Emulsion - selbstverständlich nicht beliebig groß gewählt werden, da auch die Größe der Phasen die Stabilität einer Emulsion entscheidend mit beeinflusst. Wenn eine große Ölphase (von mehr als etwa 30 Gew.%) gewünscht ist, müssen daher gemäß dem Stand der Technik Stabilisatoren wie Wachse oder weitere Emulgatoren eingesetzt werden, um eine langzeitstabile Emulsion mit einer Stabilität von mehreren Jahren zu erhalten. Nachteil dieser Vorgehensweise ist allerdings, dass die Emulsionen dadurch relativ fest werden und - ins besondere auf behaarter Haut - nicht mehr so gut zu verteilen sind.

Eine weitere, nach dem Stand der Technik bekannte Methode, Lichtschutzzubereitungen mit sehr hohen Lichtschutzfaktoren (LSF größer 25) herzustellen, besteht darin, UV-Filtersubstanzen so zu kombinieren, dass sich nicht die gesamte UV-Filtermenge in der Ölphase der Emulsion befindet, was selbstverständlich nur dann möglich ist, wenn auch wasserlösliche UV-Filtersubstanzen eingesetzt werden. Nachteil derartiger Emulsionen, welche wasserlösliche UV-Filtersubstanzen enthalten, ist, dass diese gewöhnlich nur bedingt wasserfest sind.

Der Wasserfestigkeit von Lichtschutzformulierungen ist aber eine besondere Bedeutung beizumessen, da die meisten Sonnenschutzmittel in Wassernähe oder bei sportlicher Betätigung (Schwitzen) angewendet werden. Ein wasserfestes Sonnenschutzmittel schützt den Anwender nicht nur nach dem Baden, sondern bewahrt ihn auch während des Badens vor einem Sonnenbrand. Es ist ein weit verbreiteter Irrtum, dass Wasser einen guten oder gar ausreichenden Schutz vor ultravioletter Strahlung bietet. Vielmehr haben Untersuchungen gezeigt, dass noch 1 m unter der Wasseroberfläche die Durchlässigkeit für UV-B-Strahlen bei ca. 50 % liegt. Es ist daher ratsam, dass auch Wassersportler, z. B. Schwimmer, Surfer oder Schnorcheln, sowie insbesondere Kinder, die oft stundenlang am bzw. im Wasser spielen, die Haut mit einem gut haftenden, durch (Salz-) Wasser und Schweiß nur schwer abspülbaren Sonnenprodukt vor einer zu intensiven und übermäßigen Sonneneinstrahlung schützen.

Im Sinne einer optimalen Wasserfestigkeit wäre daher der Verzicht auf wasserlösliche UV-Filter wünschenswert.

Aufgabe der vorliegenden Aufgabe war es daher nicht nur, den Stand der Technik zu bereichern und seinen Nachteilen abzuhelfen, sondern dünnflüssige W/O-Emulsionen bereit zu stellen, die bei gleichbleibender Formelstabilität einen erhöhten Lichtschutzfilteranteil besitzen.
Es war daher auch die Aufgabe der vorliegenden Erfindung, auf einfache und ggf. preiswerte Weise zu Zubereitungen zu gelange n, welche eine hohe UVA- bzw. UVB-Schutzleistung erreichen, in Form von Sonnenschutzsprays zu applizieren sind und sich gleichzeitig durch gute Wasserfestigkeit und gute Verteilbarkeit auszeichnen.

Gelöst werden die angeführten Aufgaben durch Zubereitungen gemäß dem Hauptanspruch. Gegenstand der Unteransprüche sind vorteilhafte Ausführungsformen der erfindungsgemäßen Zubereitungen. Des weiteren umfasst die Erfindung die Verwendung derartiger Zubereitungen.
Es war überraschend und darin liegt die Lösung dieser Aufgaben, dass kosmetische oder dermatologische Zubereitungen auf Basis einer dünnflüssigen W/O Emulsion umfassend mehr als 30 Gew.% Lipide, bezogen auf die Gesamtmasse der Zubereitung, wobei die Zubereitung nur einen Emulgator, PEG-30 Dipolyhydroxystearat, umfasst und die Zubereitung eine Viskosität im Bereich von 50 bis 2000 mPa s, bevorzugt 100 bis 600 mPa s, aufweist, die Aufgaben vollständig löst.

Es war überraschend und für den Fachmann nicht vorauszusehen, dass der Einsatz von PEG-30-Dipolyhydroxystearat als einziger Emulgator den Einsatz von einem hohen Anteil an Lipiden ermöglicht und diese Zubereitung dadurch erst die geforderte Dünnflüssigkeit, und insbesondere die Sprühbarkeit, ermöglicht
Die Viskosität ist bestimm bar mit einem Haake Viskotester VT-02 bei 25°C und beträgt vorteilhaft 50 bis 2000 mPa s, idealerweise 100 bis 600 mPa s.

PEG-30-Dipolyhydroxystearat ist bekannt und wird beispielsweise unter dem Handelsnamen ARLACEL® P135 verkauft. Erfindungsgemäß enthalten die Zubereitung keine weiteren Emulgatoren.
Erstaunlicherweise ist die Kombination dieses Emulgators mit einem hohen Lipidanteil und einer geringen Viskositätseinstellung entscheidend für die Bereitstellung einer dünnflüssigen Zubereitung, die die aufgezeigten Nachteile des Standes der Technik vermeiden hilft.

Die erfindungsgemäßen Zubereitungen stellen in jeglicher Hinsicht überaus befriedi - gende Präparate dar, die eine gute Formel- und Lagerstabilität aufweisen, langzeitstabil auch bei tieferen Temperaturen sind und als Lichtschutzpräparat den Einsatz von großen Mengen Lichtschutzfiltermengen ermöglichen.

Als hoher Lipidanteil gilt mehr als 30 Gew.%, insbesondere mehr als 35 Gew.%, besonders bevorzugt sogar größer 37,5 Gew.% und ganz besonders bevorzugt mehr als 40 Gew.% Lipide, jeweils bezogen auf das Gesamtgewicht der Rezeptur. Diese Aufteilung der Lipidanteile ist je nach Produktform zu wählen.

Zur Lipidphase zählen dabei erfindungsgemäß nicht der Emulgator sowie weitere Wirk-oder Zusatzstoffe, die unter Umständen lipophile Eigenschaften aufweisen können.

Im Rahmen der vorliegenden Offenbarung wird als Oberbegriff für Fette, Öle, Wachse und dergleichen der Ausdruck "Lipide" verwendet, wie dem Fachmanne durchaus geläufig ist. Auch werden die Begriffe "Ölphase'' und "Lipidphase" synonym angewandt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, dass die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar im Sinne der vorliegenden Erfindung werden Lipide angesehen, deren Grenzflächenspannung gegen Wasser weniger als 20 mN/m beträgt als unpolar solche, deren Grenzflächenspannung gegen Wasser mehr als 30 mN/m beträgt. Lipide mit einer Grenzflächenspannung gegen Wasser zwischen 20 und 30 mN/m werden im allgemeinen als mittelpolar bezeichnet.

Bevorzugt werden die Lipide gewählt aus der Gruppe der mittel- bis unpolaren Lipide mit einer Grenzflächenspannung gegen Wasser von 20 bis 30 mN/m bzw. über 30 mN/m.

Der Anteil an polaren Lipiden, mit einer Grenzflächenspannung gegen Wasser kleiner 20 mN/m, beträgt vorteilhaft weniger als 25 Gew.%, bevorzugt weniger als 15 Gew.%, bezogen auf die Gesamtmasse an Lipiden.
Polare Öle, sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.
Als Vertreter der polaren Lipide hat sich auch Cocoglycides, Cocoglycerides, erfolgreich bewährt.

Besonders vorteilhafte polare Lipide im Sinne der vorliegenden Erfindung sind alle nativen Lipide, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl und dergleichen sowie die im folgenden aufgelisteten.

| **Handelsname** | **INCl-Name** | **Polarität [mN/m]** |
|---|---|---|
| Isofol 14 T | Butyl Decanol (+) Hexyl Octanol (+) Hexyl Decanol (+) Butyl Octanol | 19,8 |
| Lipovol MOS-130 | Tridecyl Stearate(+) Tridecyl Trimellitate(+) Dipentaerythrityl Hexacaprylate/Hexacaprate | 19,4 |
| Ricinusoel | | 19,2 |
| Isofol Ester 0604 | | 19,1 |
| Miglyol 840 | Propylene Glycol Dicaprylate/Dicaprate | 18,7 |
| Isofol 12 | Butyl Octanol | 17,4 |
| Tegosoft SH | Stearyl Heptanoate | 17,8 |
| Avocadooel | | 14,5 |
| Cetiol B | Dibutyl Adipate | 14,3 |
| Dermol 488 | PEG 2 Diethylenhexanoate | 10,1 |
| Cosmacol ELI | C12-13 Alkyl Lactate | 8,8 |
| Dermol 489 | Diethylen Glycol Dioctanoate(/ Diisononanoate | 8,6 |
| Cosmacol ETI | Di-C12/13 Alkyl Tartrate | 7,1 |
| Emerest 2384 | Propylene Glycol Monoisostearate | 6,2 |
| Myritol 331 | Cocoglycerides | 5,1 |
| Prisorine 2041 GTIS | Triisostearin | 2,4 |

Ferner kann die Ölphase vorteilhaft gewählt werden aus der Gruppe der Dialkylether, der Gruppe der gesättigten oder ungesättigten, verzweigten oder unverzweigten Alkohole. Es ist insbesondere vorteilhaft, wenn die Ölphase einen Gehalt an C₁₂₋₁₅-Alkylbenzoat aufweist oder vollständig aus diesem besteht.

Ferner kann die Ölphase vorteilhaft gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung 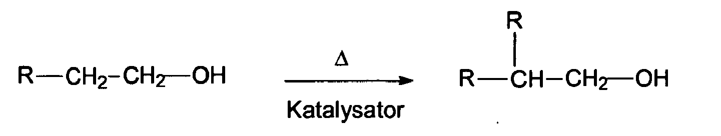 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur 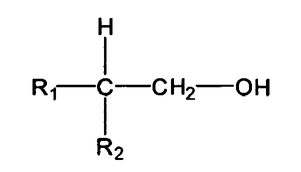 aus. Dabei bedeuten R₁ und R₂ in der Regel unverzweigte Alkylreste.

Erfindungsgemäß vorteilhaft werden der oder die Guerbet-Alkohole gewählt aus der Gruppe, bei denen
- R₁ =: Propyl, Butyl, Pentyl, Hexyl, Heptyl oder Octyl und
- R₂ =: Hexyl, Heptyl, Octyl, Nonyl, Decyl, Undecyl, Dodecyl, Tridecyl oder Tetradecyl.

Erfindungsgemäß bevorzugte Guerbet-Alkohole sind das 2-Butyloctanol - es hat die chemische Struktur 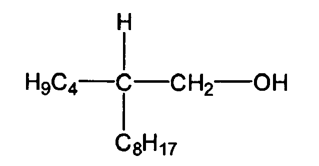 und ist beispielsweise unter der Handelsbezeichnung Isofol^{®} 12 von der Gesellschaft Condea Chemie GmbH erhältlich - und das 2-Hexyldecanol - es hat die chemische Struktur 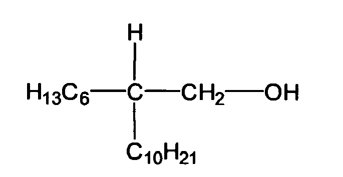 und ist beispielsweise unter der Handelsbezeichnung Isofol® 16 von der Gesellschaft Condea Chemie GmbH erhältlich. Auch Mischungen von erfindungsgemäßen Guerbet-Alkoholen sind erfindungsgemäß vorteilhaft zu verwenden. Mischungen aus 2-Butyloctanol und 2-Hexyldecanol sind beispielsweise unter der Handelsbezeichnung Isofol® 14 von der Gesellschaft Condea Chemie GmbH erhältlich.

Besonders vorteilhafte mittelpolare Lipide im Sinne der vorliegenden Erfindung sind die im folgenden aufgelisteten Substanzen:

| **Handelsname** | **INCl-Name** | **Polarität [mN/m]** |
|---|---|---|
| DUB VCl 10 | Isodecyl Neopentanoate | 29,9 |
| Dermol IHD | Isohexyldecanoate | 29,7 |
| Dermol 108 | Isodecyl Octanoate | 29,6 |
| Dihexyl Ether | Dihexyl Ether | 29,2 |
| Dermol 109 | Isodecyl 3,5,5 Trimethyl Hexanoate | 29,1 |
| Cetiol SN | Cetearyl Isononanoate | 28,6 |
| Isopropylpalmitat | Isopropylpalmitat | 28,8 |
| DC Fluid 345 | Cyclomethicone | 28,5 |
| Dow Corning Fluid 244 | Cyclopolydimethylsiloxan | 28,5 |
| Jojobaöl Gold | | 26,2 |
| Wacker AK 100 | Dimethicone | 26,9 |
| Dermol 98 | 2- Ethylhexanosäure 3,5,5 Trimethylester | 26,2 |
| Dow Corning Fluid 246 | Offen | 25,3 |
| Eutanol G | Octyldodecanol | 24,8 |
| Isofol 16 | Hexyl Decanol | 24,3 |
| Dermol 139 | Isotridecyl 3,5,5 Trimethylhexanonanoate | 24,5 |
| *Cetiol PGL* | Hexyldecanol (+) Hexyl Decyl Laurate | 24,3 |
| Cegesoft C24 | Octyl Palmitate | 23,1 |
| M.O.D. | Octyldodeceyl Myristate | 22,1 |
| Macadamia Nut Oil | | 22,1 |
| *Silikonöl VP 1120* | Phenyl Trimethicone | 22,7 |
| Isocarb 12 | Butyl Octanoicacid | 22,1 |
| Isopropylstearat | Isopropyl Stearate | 21,9 |
| Finsolv TN | C12-15 Alkyl Benzoate | 21,8 |
| Dermofeel BGC | Butylene Glycol Caprylate/Caprate | 21,5 |
| Miglyol 812 | Caprylic/Capric Triglyceride | 21,3 |
| Trivent OCG | Tricaprylin | 20,2 |
| Dermol 866 | PEG "Diethylhexanoate/ Diisononanoate/ Ethylhexyl Isononanoate | 20,1 |

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Besonders vorteilhafte unpolare Lipide im Sinne der vorliegenden Erfindung sind die im folgenden aufgelisteten Substanzen:

| **Handelsname** | **INCl-Name** | **Polarität [mN/m]** |
|---|---|---|
| Ecolane 130 | Cycloparaffin | 49,1 |
| Nexbase 2006 FG | Polydecene | 46.7 |
| Polysynlane | Hydrogenated Polyisobutene | 44.7 |
| Wacker Silikonöl AK 50 | Polydimethylsiloxan | 46,5 |
| Solvent ICH | Isohexadecane | 43.8 |
| Pionier 2076 | Mineral Oil | 43.7 |
| Pionier 6301 | Mineral Oil | 43.7 |
| Wacker Silikonöl AK 35 | Polydimethylsiloxan | 42,4 |
| Isoeikosan | Isoeikosan | 41.9 |
| Wacker Silikonöl AK 20 | Polydimethylsiloxan | 40,9 |
| Isofol 1212 Carbonat | | 40,3 |
| Softcutol O | Ethoxydiglycol Oleate | 40,5 |
| Lipodermanol OL | Decyl Olivate | 40,3 |
| *Cetiol* S | Dioctylcyclohexane | 39,0 |
| Pionier 2071 | Mineral Oil | 38.3 |
| Hydrobrite 1000 PO | Paraffinum Liquidum | 37,6 |
| Tegosoft HP | Isocetyl Palmitate | 36,2 |
| Isofol Ester 1693 | | 33,5 |
| Isofol Ester 1260 | | 33,0 |
| Dow Corning Fluid 245 | Cyclopentasiloxan | 32,3 |
| Prisorine 2036 | Octyl Isostearate | 31.6 |
| Cetiol CC | Dicaprylyl Carbonate | 31,7 |
| Dermol 99 | Trimethylhexyl Isononanoate | 31,1 |
| Dermol 89 | 2- Ethylhexyl Isononanoate | 31,0 |
| Cetiol OE | Dicaprylyl Ether | 30,9 |
| *Dihexylcarbonat* | Dihexyl Carbonate | 30,9 |
| Silkflo 366 NF | Polydecene | 30,1 |
| Estol 1540 EHC | Octyl Cocoate | 30.0 |

Es ist jedoch auch vorteilhaft Gemische aus höher- und niederpolaren Lipiden zu verwenden.
Bevorzugt werden die Lipide gewählt aus der Gruppe Dicaprylyl Carbonate, Butylene Glycol Dicaprylate/Dicaprate und/oder C12-15 Alkyl Benzoate.
Auch kann die Ölphase gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr, sofern die im Haupt- bzw. Nebenansprüchen geforderten Bedingungen eingehalten werden.

Auch Wachse können eingesetzt werden, allerdings bis maximal 3,0 Gew. %, bevorzugt bis maximal 1,5 % bezogen auf das Gesamtgewicht der Zubereitung.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montan-wachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die in den Ansprüchen geforderten Bedingungen eingehalten werden.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), und Syncrowax AW 1C (C₁₈₋₃₆ -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀-Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan sofern die in den jeweiligen Ansprüchen geforderten Bedingungen eingehalten werden.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, C₁₂₋₁₅-Alkylbenzoat, Capryl-Caprinsäuretriglycerid, Dicaprylylether, Dicaprylyl Carbonate, und Cococlyceriden, sofern die in den Ansprüchen geforderten Bedingungen eingehalten werden.

Besonders vorteilhaft sind Mischungen aus Octyldodecanol, Capryl-Caprinsäuretriglycerid, Dicaprylylether, Dicaprylyl Carbonat, Cocoglyceriden, oder Mischungen aus C₁₂₋₁₅-Alkybenzoat und 2-Ethylhexylisostearat, Mischungen aus C₁₂₋₁₅-Alkybenzoat und Butylen Glycol Dicaprylat/Dicaprat sowie Mischungen aus C₁₂₋₁₅-Alkybenzoat, 2-Ethylhexylisostearat und Isotridecylisononanoat sofern die in den jeweiligen Ansprüchen geforderten Bedingungen eingehalten werden.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden. sofern die in den Ansprüchen geforderten Bedingungen eingehalten werden.

Es kann ebenfalls vorteilhaft sein, die Ölphase der erfindungsgemäßen Zubereitungen teilweise aus der Gruppe der cyclischen und/oder linearen Silicone zu wählen. Der Anteil an cyclischen und/oder linearen Silikonen ist auf maximal 25 Gew.%, vorzugsweise auf maximal 15 Gew.% bezogen auf den Anteil an der Gesamtlipidphase, zu begrenzen. Solche Silicone oder Siliconöle können als Monomere vorliegen, welche in der Regel durch Strukturelemente charakterisiert sind, wie folgt: 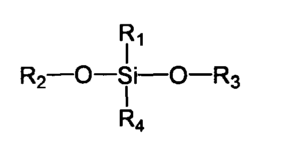 Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind.

Als erfindungsgemäß vorteilhaft einzusetzenden linearen Silicone mit mehreren Siloxyleinheiten werden im allgemeinen durch Strukturelemente charakterisiert wie folgt: 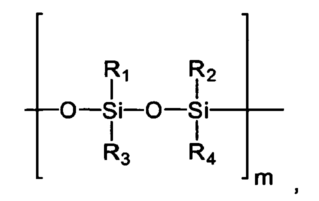 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). m kann dabei Werte von 2 - 200.000 annehmen.

Systematisch werden die linearen Silikonöle als Polyorganosiloxane bezeichnet; die methylsubstituierten Polyorganosiloxane, welche die mengenmäßig bedeutendsten Verbindungen dieser Gruppe darstellen und sich durch die folgende Strukturformel auszeichnen 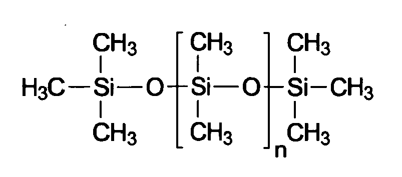 werden auch als Polydimethylsiloxan bzw. Dimethicon (INCI) bezeichnet. Dimethicone gibt es in verschiedenen Kettenlängen bzw. mit verschiedenen Molekulargewichten. Dimethicone unterschiedlicher Kettenlänge und Phenyltrimethicone sind besonders vorteilhafte lineare Silikonöle im Sinne der vorliegenden Erfindung.

Besonders vorteilhafte Polyorganosiloxane im Sinne der vorliegenden Erfindung sind ferner beispielsweise Dimethylpolysiloxane [Poly(dimethylsiloxan)], welche z. B. unter den Handelsbezeichnungen ABIL 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silicone (Octamethylcyclotetrasiloxan bzw. Decamethylcyclopentasiloxan), welche nach INCI auch als Cyclomethicone bezeichnet werden, aminomodifizierte Silicone (INCI: Amodimethicone) und Siliconwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen erhältlich sind.

Besonders vorteilhaft im Sinne der vorliegenden Erfindung sind ferner die im folgenden aufgelisteten Silikonöle:

| **Handelsname** | **INCl-Name** | **Polarität** |
|---|---|---|
| | | **[mN/m]** |
| Wacker Silikonöl AK 100 | Polydimethylsiloxan | 26,9 |
| Wacker Silikonöl AK 50 | Polydimethylsiloxan | 46,5 |
| Wacker Silikonöl AK 35 | Polydimethylsiloxan | 42,4 |
| Wacker Silikonöl AK 20 | Polydimethylsiloxan | 40,9 |
| Dow Corning Fluid 245 | Cyclopentasiloxan | 32,3 |
| Dow Corning Fluid 345 | Cyclomethicone | 28,5 |

Erfindungsgemäß vorteilhaft einzusetzende cyclische Silicone werden im allgemeinen durch Strukturelemente charakterisiert, wie folgt 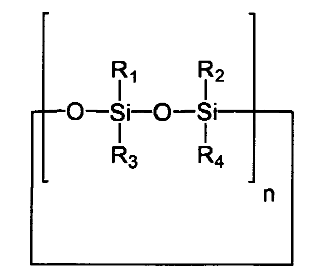 wobei die Siliciumatome mit gleichen oder unterschiedlichen Alkylresten und/oder Arylresten substituiert werden können, welche hier verallgemeinernd durch die Reste R₁ - R₄ dargestellt sind (die Anzahl der unterschiedlichen Reste sind nicht notwendig auf bis zu 4 beschränkt). n kann dabei Werte von 3/2 bis 20 annehmen. Gebrochene Werte für n beiücksichtigen, dass ungeradzahlige Anzahlen von Siloxylgruppen im Cyclus vorhanden sein können.

Besonders vorteilhafte cyclische Silikonöle im Sinne der vorliegenden Erfindung sind Cyclomethicone, insbesondere Cyclomethicone D5 und/oder Cyclomethicone D6, insbesondere auch eine Mischung aus D4, D5 und D6.

Vorteilhafte Silkonöle bzw. Silikonwachse im Sinne der vorliegenden Erfindung sind cyclische und/oder lineare Silikonöle und Silikonwachse.
Es ist besonders vorteilhaft im Sinne der vorliegenden Erfindung das Verhältnis von Lipiden zu Silikonölen in etwa wie 1 : 1 (allgemein x : y) zu wählen.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol sowie das Cetyl-Dimethicon-Copolyol (und) Polyglyceryl-4-lsostearat (und) Hexyllaurat.

Der Anteil an Lipiden umfasst erfindungsgemäß weitmehr als 30 Gew.%, bezogen auf die Gesamtmasse der Zubereitung. Das Phasenverhältnis zwischen Öl- und Wasserphase stellt im Bereich der Wasser-in-Öl Emulsionen eine entscheidende Größe dar, um den Viskositätsbereich von den hier aufgeführten Zubereitungen einzustellen: Je kleiner der Anteil der inneren dispersen Wasserphase (und entsprechend je größer der Anteil an äußerer kontinuierlichen Lipidphase) ist, desto niedrigere Viskositätsbereiche lassen sich aufgrund der reduzierten Wechselwirkung der inneren dispersen Wasserphase einstellen. Aufgrund der großen Abstände der Wassertröpfchen voneinander kann ohne Verwendung von Ölverdickern bzw. Wachsen kein Gelgerüst und damit auch keine Viskosität aufgebaut werden. Traditionelle Systeme werden aufgrund dieser Eigenschaft sehr schnell instabil (d.h. binnen Tages- bzw. Wochenfrist, insbesondere bei hohen Lager-temperaturen von 40°C und mehr).
Bevorzugt liegt das Phasenverhältnis zwischen Öl- und Wasserphase im Bereich von 30 zu 70 bis 60 zu 40, insbesondere 35:65 bis 50:50, besonders bevorzugt 40:60. Hierbei zählt der Emulgator und ggf. weitere lipophile Verbindungen nicht zur Ölphase hinzu.

Erfindungsgemäß wird dieses Problem der Dünnflüssigkeit, Sprühbarkeit und gleichzeitiger Stabilität durch die erfindungsgemäße Zubereitung gelöst.
Dementsprechend eignen sich Zubereitungen im Sinne der vorliegenden Erfindung ganz besonders, um als Grundlage für Produktformen mit vielfältigen Anwendungszwecken zu dienen.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Butylen Glykol, Ethylenglykol, Ethylhexylglycerin, Ethylenglykolmonoethyl- oder -monobutylether, Propylenglykolmonomethyl, -monoethyl- oder -monobutylether, Diethylenglykolmonomethyl- oder - monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Vorteilhafte Konservierungsmittel im Sinne der vorliegenden Erfindung sind beispielsweise Formaldehydabspalter (wie z. B. DMDM Hydantoin [z.B. Glydant®]), lodopropylbutylcarbamat (z. B. unter den Handelsbezeichnungen Koncyl-L, Koncyl-S und Konkaben LMB), Parabene, Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Ethylhexyloxyglycerin, Glycine Soja etc.

Des weitern können Feuchthaltemittel bzw. sogenannte Moisturizer enthalten sein. Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registratumummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

Die erfindungsgemäßen Zusammensetzungen können ferner gegebenenfalls in der Kosmetik übliche Zusatzstoffe, beispielsweise Parfüm, Verdicker, Desodorantien, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate enthalten.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch.
Vorteilhafte Farbpigmente sind beispielsweise Titandioxid, Glimmer, Eisenoxide (z. B. Fe₂O₃, Fe₃O₄, FeO(OH)) und/oder Zinnoxid.
Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:
Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.% bis 30 Gew.%, vorzugsweise von 0,5 bis 15 Gew.%, insbesondere von 1,0 bis 10 Gew.% gewählt, jeweils bezogen auf das Gesamtgewicht der Zubereitungen.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Camosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ -Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg - Ascorbylphosphat, Ascorbylacetat), Tocopherole und Derivate (z. B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Eine erstaunliche Eigenschaft der erfindungsgemäßen Zubereitungen ist, dass diese sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.%, besonders bevorzugt 0,05 bis 20 Gew.%, insbesondere 0,1 bis 10 Gew.%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:
Acetylsalicylsäure, Atropin, Azulen, Hydrocortison und dessen Derivaten, z. B. Hydrocortison-17-valerat, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die gamma-Linolensäure, Ölsäure, Eicosapentaensäure, Docosahexaensäure und deren Derivate, Chloramphenicol, Coffein, Prostaglandine, Thymol, Campher, Extrakte oder andere Produkte pflanzlicher und tierischer Herkunft, z. B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit^{®} und Neocerit^{®}.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspositionen angegeben):

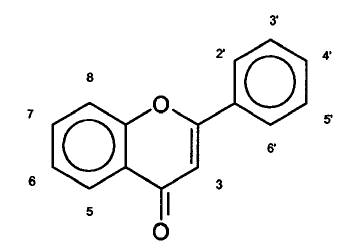

Einige der wichtigeren Flavone, welche auch bevorzugt in erfindungsgemäßen Zubereitungen eingesetzt werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt aus der Gruppe der Substanzen der generischen Strukturformel 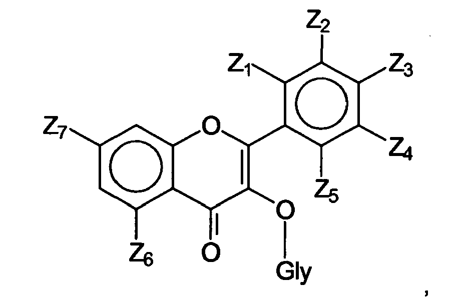 wobei Z₁ bis Z₇ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 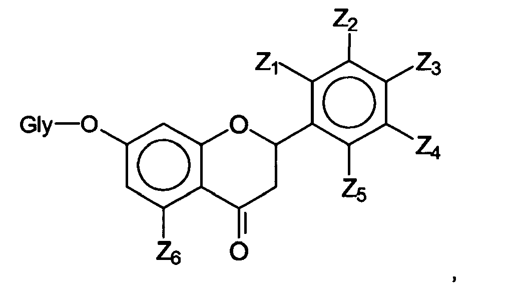 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 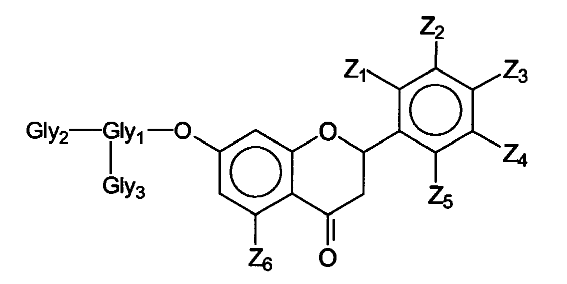 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur

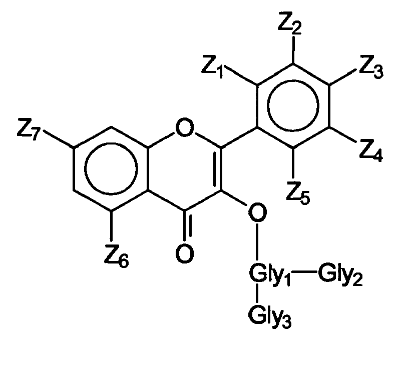

Besonders vorteilhaft werden die erfindungsgemäßen Flavonglycoside aus der Gruppe, welche durch die folgende Struktur wiedergegeben werden: 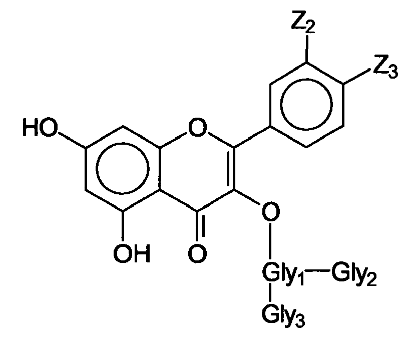 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, dem oder die Wirkstoffe aus der Gruppe der Ubichinone und Plastochinone zu wählen.

### Ubichinone zeichnen sich durch die Strukturformel

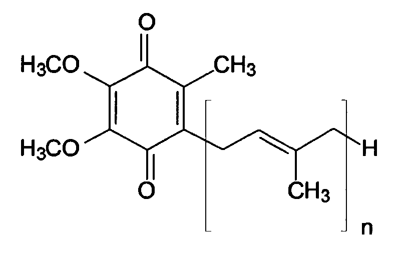

aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

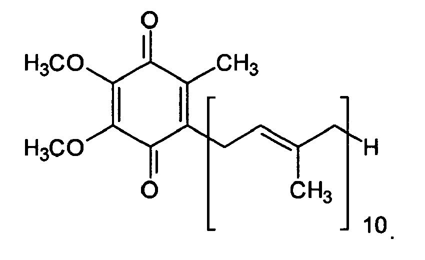

### Plastochinone weisen die allgemeine Strukturformel

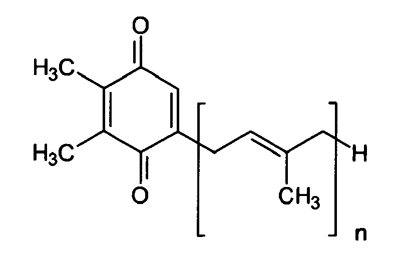

auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung. Kreatin zeichnet sich durch folgende Struktur aus:

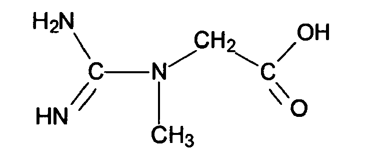

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist L-Carnitin [3-Hydroxy-4-(trimethylammonio)-butter-säurebetain]. Auch Acyl-Carnitine, welche gewählt aus der Gruppe der Substanzen der folgenden allgemeinen Strukturformel 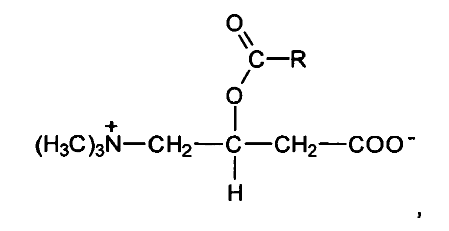 wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Wirkstoffe sind Sericosid, Pyridoxol, Vitamin K, Biotin und Aromastoffe.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Darüber hinaus eignen sich ausgewählte erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, insbesondere vorteilhaft zur Prophylaxe und Behandlung kosmetischer oder dermatologischer Hautveränderungen, wie sie z. B. bei der Hautalterung auftreten. Weiterhin vorteilhaft eignen sie sich gegen das Erscheinungsbild der trockenen bzw. rauhen Haut.

Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:
a) Trockenheit, Rauhigkeit und Ausbildung von (Trockenheits-) Fältchen,
b) Juckreiz und
c) verminderte Rückfettung durch Talgdrüsen (z. B. nach dem Waschen).
   Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

In einer besonderen Ausführungsform betrifft die vorliegende Erfindung insbesondere Produkte zur Pflege der auf natürliche Weise gealterten Haut, sowie zur Behandlung der Folgeschäden der Lichtalterung, insbesondere der unter a) bis g) aufgeführten Phänomene.

Die erfindungsgemäßen kosmetischen und/oder dermatologischen Zubereitungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Aufgrund der Dünnflüssigkeit im beanspruchten Viskositätsbereich und aufgrund der besonderen Produkteigenschaften kann insbesondere die Applikation auf die Haut durch Aufsprühen der Zubereitungen erfolgen. Das Versprühen kann beispielsweise mittels einfacher Pump-Zerstäuber, Handzerstäuberpumpen mit Einfinger- oder "Trigger-Betätigung", durch Aerosolpumpen, bei denen der Austrag des Füllguts durch ein Treibmittel erfolgt, durch Dispenserpumpen oder Quetschflaschen oder durch sog. Bag-in-Can Systeme erfolgen.
Die erfindungsgemäßen Zubereitungen lassen sich mit den standardmäßigen Pumpen problemlos applizieren und erzeugen ein feines und gleichmäßiges Sprühbild.

Eine weitere vorteilhafte Ausführungsform der vorliegenden Erfindung besteht in Sonnenschutz-Produkten.
Besonders vorteilhaft ist ein Zusatz von öllöslichen und/oder wasserlöslichen UV-Filtern und/oder UV-Strahlung absorbierender bzw. reflektierender anorganischer Pigmente.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden, sowie das Sulfat des Bariums (BaSO₄).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form commerziell erhältlicher öliger oder wäßriger Vordispersionen zur Anwendung kommen. Diesen Vordispersionen können vorteilhaft Dispergierhilfmittel und/oder Solubilisationsvermittler zugesetzt sein.
Die erfindungsgemäßen Titandioxide zeichnen sich durch eine Primärpartikelgröße zwischen 10 nm bis 150 nm aus.

| **Handelsname** | **Coating** | **zusätzliche Bestandteile der Vordispersion** | **Hersteller** |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KgaA |
| Eosolex TS | Alumina, Stearinsäure | - | Merck KgaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wäßriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Die Gesamtmenge an einem oder mehreren anorganischen Pigmenten in der fertigen kosmetischen Zubereitung wird vorteilhaft aus dem Bereich 0,1 Gew.% bis 25 Gew.% gewählt, vorzugsweise 0,5 Gew.% bis 18 Gew.%.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel 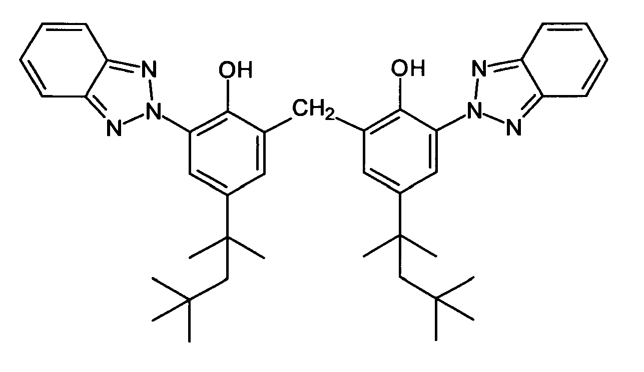 gekennzeichnet ist und unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure 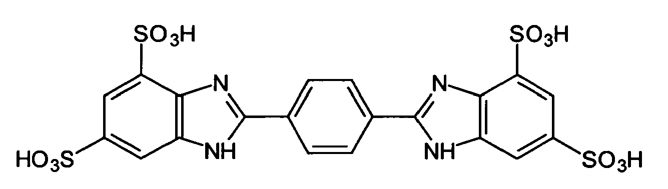 und ihre Salze, besonders die entsprechenden Natrium-, Kalium- o der Triethanolammonium-Salze, insbesondere das Phenylen-1,4-bis-(2-benzimidazyl)3,3'-5,5'-tetrasulfonsäure-bis-natriumsalz 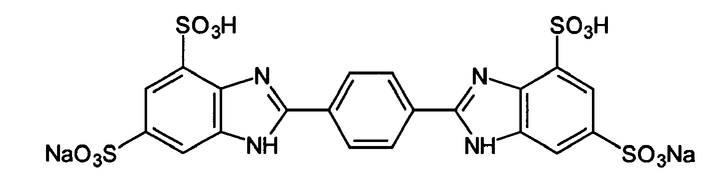 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

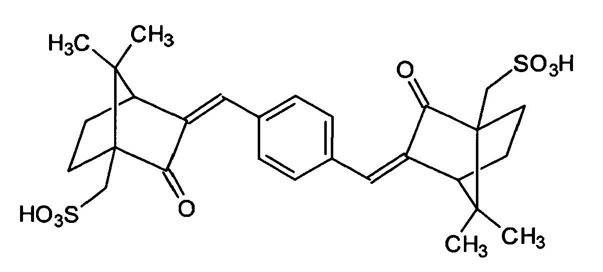

Weitere vorteilhafte UV-A-Filtersubstanzen sind Hydroxybenzophenone, die sich durch die folgende Strukturformel auszeichnen: 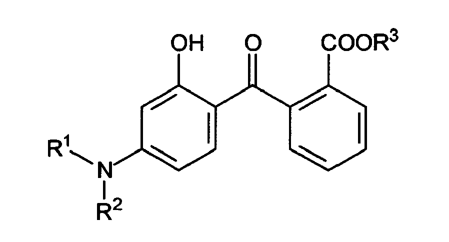 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 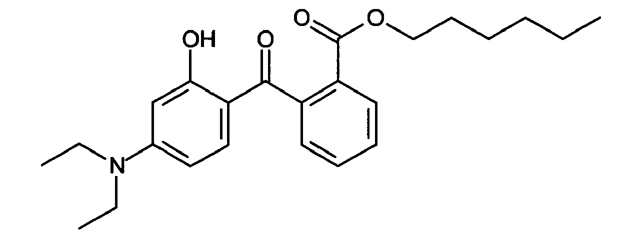 und unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 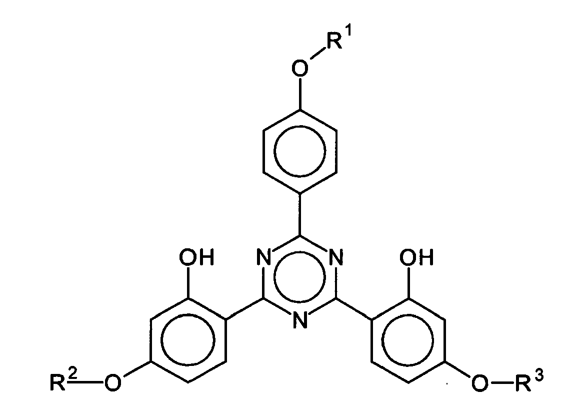 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethylhexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

### Auch andere UV-Filtersubstanzen, welche das Strukturmotiv

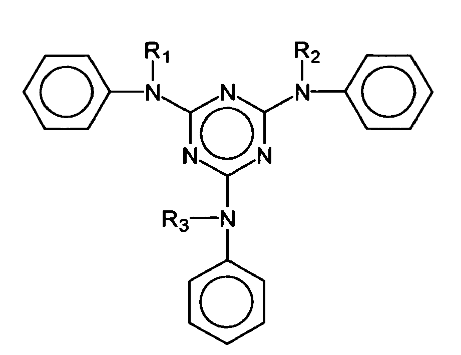

aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 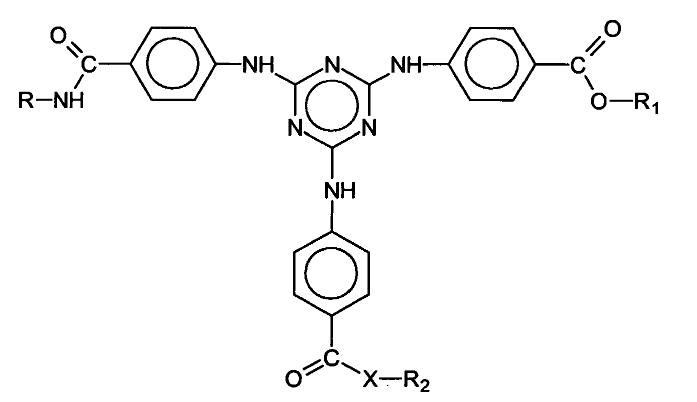 wiedergegeben wird, wobei
- R: einen verzweigten oder unverzweigten C₁-C₁₈-Alkylrest, einen C₅-C₁₂-Cycloalkyl-rest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkyl-rest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 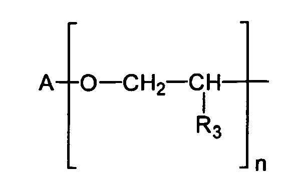
bedeutet, in welcher
- A: einen verzweigten oder unverzweigten C₁-C₁₈-Alkylrest, einen C₅-C₁₂-Cyclo-alkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 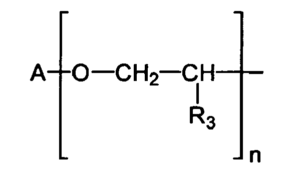
bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 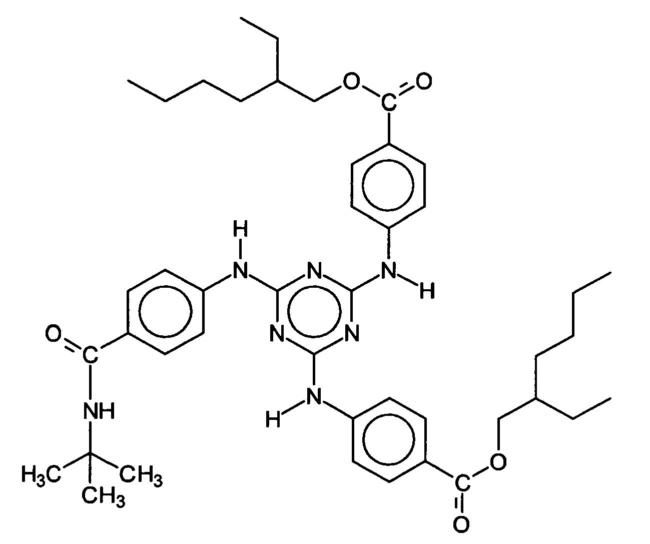 wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoësäure-tris(2-ethylhexyl-ester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 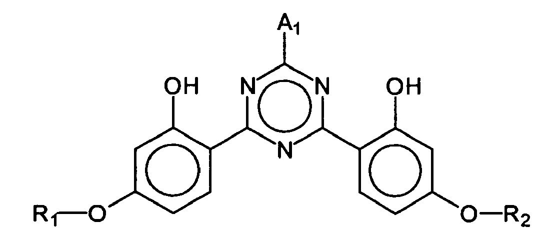 wiedergegeben wird, wobei R₁, R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.
Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 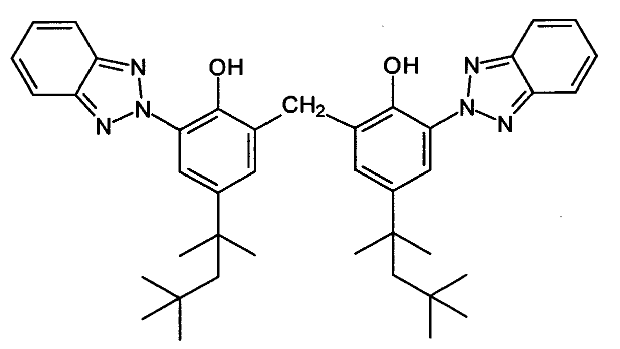 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 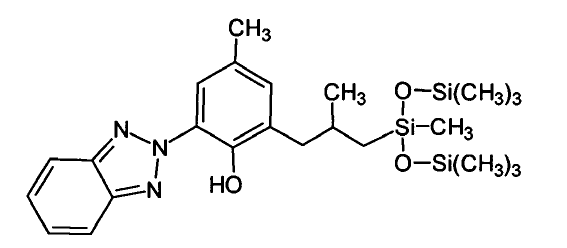 gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-hydroxybenzoat (2-Ethylhexylsalicylat, Octylsalicylat, INCI: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/ Dimethylsiloxan - Copolymer (INCI: Dimethicodiethylbenzalmalonat) welches beispielsweise unter der Handelsbezeichnung Parsol® SLX bei Hoffmann La Roche erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist und sich durch folgende Struktur auszeichnet: 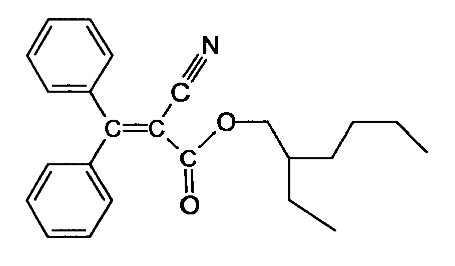 Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Liste der genannten UV-Filter, die im Sinne der vorliegenden Erfindung eingesetzt werden können, soll selbstverständlich nicht limitierend sein

Hier sind sowohl hohe UVA- als auch UVB-Filtermengen in der erfindungsgemäßen Zubereitung möglich. Zu nennen sind insbesondere die UV-Filter Ethylhexyl Methoxycinnamate (Parsol MCX), Phenylbenzimidazole Sulfonic Acid (Eusolex 232), Ethylhexyl Triazone (Uvinul T150), Bis-Ethylhexyloxyphenol Methoxyphenyl Triazine sowie Butyl methoxydibenzoylmethane (Parsol 1789) und Titandioxid.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.% bis 30 Gew.%, vorzugsweise 0,5 bis 20 Gew.%, insbesondere 1,0 bis 15,0 Gew.%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.
Die erfindungsgemäßen Zubereitungen stellen in jeglicher Hinsicht überaus befriedigende Präparate dar, welche sehr gute sensorische und kosmetische Eigenschaften zeigen, wie beispielsweise die Verteilbarkeit auf der Haut oder das Einzugsvermögen in die Haut. Sie zeichnen sich ferner durch eine sehr gute Lichtschutzeffektivität, eine überaus hohe UV-A-Schutzleistung sowie durch eine ausgezeichnete Hautverträglichkeit bei gleichzeitig hervorragenden Hautpflegedaten aus.
Vorteil der vorliegenden Erfindung ist, dass trotz eines hohen Ölphasenanteiles von mehr als 30 Gew.%, bezogen auf das Gesamtgewicht der Zubereitung, kein weiterer Emulgator bzw. Coemulgator notwendig ist.
Zudem, und das ist eine weiterer erfindungsgemäße Erkenntnis, lassen sich hohe Anteile an Lichtschutzfiltern, insbesondere UVA- und/oder UVB-Filter, in die erfindunsggemäße Zubereitung einbauen ohne dass die Stabilität oder die Viskosität Einbußen erfahren. Ein sehr hoher Anteil an Lichtschutzfiltern bedeutet je nach Lichtschutzfaktor zwischen 15 und 25 Gewichtsprozent, bezogen auf die Gesamtmasse der Zubereitung.

Zudem liegt vorteilhafterweise ein sehr hoher Gehalt an Lipiden vor von über 35 Gew.%. Als besonders bevorzugt hat sich daher eine Zubereitung herausgestellt, die 17 - 23 Gew.%, bevorzugt 20 Gew.%, Lichtschutzfilter, 35 - 45 Gew.%, bevorzugt 40 Gew.%, Lipide und 2 - 7 Gew.%, bevorzugt 5 Gew.%, Emulgator enthält, jeweils bezogen auf die Gesamtmasse der Zubereitung.

Ferner kann es gegebenenfalls von Vorteil sein, Filmbildner in die erfindungsgemäßen kosmetischen oder dermatologischen Zubereitungen einzuarbeiten, beispielsweise um die Wasserfestigkeit der Zubereitungen zu verbessern oder die UV-Schutzleistung zu erhöhen (UV-A- und /oder UV-B-Boosting). Geeignet sind sowohl wasserlösliche bzw. dispergierbare als auch fettlösliche Filmbildner, jeweils einzeln oder in Kombination miteinander.

Vorteilhafte wasserlöslich bzw. dispergierbare Filmbildner sind z. B. Polyurethane (z. B. die Avalure® -Typen von Goodrich), Dimethicone Copolyol Polyacrylate (Silsoft Surface® von der Witco Organo Silicones Group), PVP/VA (VA = Vinylacetat) Copolymer (Luviscol VA 64 Powder der BASF) etc.

Vorteilhafte fettlösliche Filmbildner sind z. B., die Filmbildner aus der Gruppe der Polymere auf Basis von Polyvinylpyrrolidon (PVP) 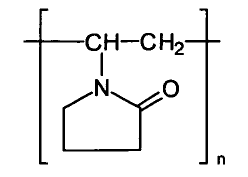 Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Erfindungsgemäß können diese Emulsionen als kosmetische und dermatologische Zubereitungen auch als Reinigungsmittel eingesetzt werden.

Kosmetische Zubereitungen, die kosmetische Reinigungszubereitungen für die Haut darstellen, können in flüssiger oder fester Form vorliegen. Sie enthalten neben erfindungsgemäßen Wirkstoffkombinationen vorzugsweise mindestens eine anionische, nicht-ionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.% in den Reinigungszubereitungen vorliegen, bezogen auf das Gesamtgewicht der Zubereitungen.

Eine weitere vorteilhafte Ausführungsform der vorliegenden Erfindung besteht in insektenabweisenden Mitteln.

Vorteilhafte Wirkstoffe für Repellents sind niedrig schmelzende oder flüssige Amide, Alkohole Ester und Ether mit Schmelzpunkten über 150°C, die bei Raumtemperatur nur langsam Verdampfen.

Als besonders vorteilhaft haben sich folgende Wirkstoffe einzeln oder in Kombination miteinander oder mit anderen erwiesen: 3-(N-n-Butyl-N-acetyl-amino)-propionsäureethylester (Handelname: Insekt-Repellent 3535 bei der Fa. Merck erhältlich), N,N-Diethyl-3-methylbenzamid (DEET), Dimethylphthalat, Ethylhexandiol, Caprylsäurediethylamid und natürliche Pflanzeöle wie Citronellöl, Eucalyptusöl, Lavendelöl, Nelkenöl.

Eine weitere vorteilhafte Ausführungsform der vorliegenden Erfindung besteht in Selbstbräunern.

Vorteilhafte Wirkstoffe für Selbstbräuner sind natürliche oder synthetische Ketole oder Aldole.
Als vorteilhaft haben sich Dihydroxyaceton (DHA), Glycerolaldehyd, Erythrulose, Alloxan, Hydroxymethylglyoxal, γ-Dialdehyd, 6-Aldo-D-Fructose, Ninhydrin und meso-Weinsäuredialdehyd erwiesen.

Als besonders vorteilhaft habe sich Mischungen der o.g. Wirkstoffe untereinander oder mit Mucondialdehyd oder/und Naphthochinone wie z.B. 5-Hydroxy-1,4-naphthochinon (Juglon) und 2-Hydroxy-1,4-Naphthochinon
Weitere vorteilhafte Selbstbräunungsmittel sind 4-fach substituierte Cyclohexen-Verbindungen, insbesondere 9-Retinal-alkanolamin Schiffsche Base, wie sie in DE 10212865 beschrieben sind, die hiermit zum Offenbarungsgehalt der vorliegenden Erfindung zählt.

### Beispielrezepturen

Die nachfolgenden Beispiele sollen die vorliegende Erfin dung verdeutlichen, ohne sie einzuschränken. Die Zahlenwerte in den Beispielen bedeuten Gewichtsprozente, bezogen auf das Gesamtgewicht der jeweiligen Zubereitungen.

| **Beispiel** | **1** | **2** | **3** | **4** | **5** |
|---|---|---|---|---|---|
| Methylparaben | 0 | 0 | 0,3 | 0,3 | 0,3 |
| Propylparaben | 0 | 0 | 0,15 | 0,15 | 0,15 |
| Aqua | Ad 100 | Ad 100 | Ad 100 | Ad 100 | Ad 100 |
| Magnesium Sulfate | 0,5 | 0,5 | 0,5 | 0,5 | 0,5 |
| Citric Acid | 0,086 | 0,086 | 0 | 0 | 0 |
| Glycerin | 10 | 5 | 5 | 15 | 3 |
| Tocopheryl Acetate | 0,5 | 0,5 | 0,5 | 0,5 | 0,5 |
| Trisodium EDTA | 1 | 1 | 1 | 1 | 1 |
| Ethylhexyl Methoxycinnamate + BHT | 9 | 9 | 9 | 9 | 9 |
| C12-15 Alkyl Benzoate | 9,5 | 9,5 | 9,5 | 9,5 | 9,5 |
| Butyl Methoxydibenzoylmethane | 2 | 1 | 1 | 0,5 | 1 |
| Phenylbenzimidazole Sulfonic Acid | 0 | 0 | 4 | 4 | 4 |
| Sodium Citrate | 0,174 | 0,174 | 0 | 0 | 0 |
| Sodium Hydroxide | 0 | 0 | 0 | 0 | 0 |
| Alcohol Denat. | 3 | 0 | 0 | 0 | 0 |
| Glycine | 0,5 | 0,5 | 0,5 | 0,5 | 0,5 |
| Ethylhexyl Triazone | 3 | 3 | 3 | 3 | 3 |
| Cl 77891 + Trimethoxycaprylylsilane | 2 | 2 | 2 | 2 | 2 |
| Ethylhexylglycerin | 0,5 | 0,5 | 0,5 | 0,5 | 0,5 |
| PEG-30 Dipolyhydroxystearate | 5 | 5 | 3 | 6 | 7 |
| Bis-Ethylhexyloxyphenol Methoxyphenyl Triazine | 2 | 2 | 2 | 2 | 2 |
| Cetyl Dimethicone | 0,5 | 0,5 | 0,5 | 0,5 | 0,5 |
| Butylene Glycol Dicaprylate/Dicaprate | 9,5 | 9,5 | 9,5 | 9,5 | 9,5 |
| Cocoglycerides | 10 | 5 | 10 | 15 | 10 |
| Potassium Sorbate | 0,067 | 0,067 | 0 | 0 | 0 |
| Parfum | 0,2 | 0,2 | 0,2 | 0,2 | 0,2 |
| Dicaprylyl Carbonate | 10 | 15 | 10 | 15 | 10 |

## Patentansprüche

1. Kosmetische oder dermatologische Zubereitung auf Basis einer dünnflüssigen W/O Emulsion umfassend mehr als 30 Gew.% Lipide, bezogen auf die Gesamtmasse der Zubereitung, **dadurch gekennzeichnet, dass** die Zubereitung nur einen Emulgator, PEG-30 Dipolyhydroxystearat, umfasst und die Zubereitung eine Viskosität im Bereich von 50 bis 2000 mPa s, bevorzugt 100 bis 600 mPa s, aufweist.

2. Zubereitung nach Anspruch 1, **dadurch gekennzeichnet, dass** der Lipidanteil mehr als 35 Gew.%, besonders bevorzugt größer 37,5 Gew.% und ganz besonders bevorzugt mehr als 40 Gew.% beträgt, jeweils bezogen auf das Gesamtgewicht der Zubereitung, wobei Emulgatoren, Lichtschutzfilter und sonstige Wirk- oder Hilfsstoffe nicht zur Lipidphase der Zubereitung zählen.

3. Zubereitung nach Anspruch 1 oder 2, **dadurch gekennzeichnet, dass** die Lipide gewählt werden aus der Gruppe der mittel- bis unpolaren Lipide mit einer Grenzflächenspannung gegen Wasser von 20 bis 30 mN/m bzw. über 30 mN/m.

4. Zubereitung nach einem der vorstehenden Ansprüche, **dadurch gekennzeichnet, dass** als Lipide gewählt werden Dicaprylyl Carbonate, Butylene Glycol Dicaprylate/Dicaprate und/oder C12-15 Alkyl Benzoate.

5. Zubereitung nach einem der vorstehenden Ansprüche, **dadurch gekennzeichnet, dass** der Anteil an polaren Lipiden, mit einer Grenzflächenspannung gegen Wasser kleiner 20 mN/m, weniger als 25 Gew.%, bevorzugt weniger als 15 Gew.%, bezogen auf die Gesamtmasse an Lipiden, beträgt.

6. Zubereitung nach einem der vorstehenden Ansprüche, **dadurch gekennzeichnet, dass** das Phasenverhältnis zwischen Öl- und Wasserphase im Bereich von 30 zu 70 bis 60 zu 40, insbesondere 35:65 bis 50:50, besonders bevorzugt 40:60 beträgt, wobei Emulgatoren, Lichtschutzfilter und sonstige Wirk- oder Hilfsstoffe nicht zur Lipidphase der Zubereitung zählen.

7. Zubereitung nach einem der vorstehenden Ansprüche, **dadurch gekennzeichnet, dass** zusätzlich ein oder mehrer Lichtschutzfilter, insbesondere UVA- und/oder UVB-Filter, enthalten sind.

8. Zubereitung nach Anspruch 7, **dadurch gekennzeichnet, dass** als Lichtschutzfilter Ethylhexyl Methoxycinnamate, Phenylbenzimidazole Sulfonic Acid, Ethylhexyl Triazone, Bis-Ethylhexyloxyphenol Methoxyphenyl Triazine, Butylmethoxydibenzoylmethane und/oder Titandioxid gewählt werden.

9. Zubereitung nach Anspruch 7 oder 8, **dadurch gekennzeichnet, dass** der Anteil an Lichtschutzfiltern bis zu 25 Gew.%, bevorzugt zwischen 17 und 23 Gew.%, bezogen auf die Gesamtmasse der Zubereitung, beträgt.

10. Zubereitung nach Anspruch 7, 8 oder 9, **dadurch gekennzeichnet, dass**
- 17 - 23 Gew.%, bevorzugt 20 Gew.%, Lichtschutzfilter,
- 35 - 45 Gew.%, bevorzugt 40 Gew.%, Lipide und
- 2 - 7 Gew.%, bevorzugt 5 Gew.%, Emulgator enthalten sind, jeweils bezogen auf die Gesamtmasse der Zubereitung.

11. Zubereitung nach einem der vorhergehenden Ansprüche, **dadurch gekennzeichnet, dass** sie ein oder mehrere zusätzliche Zusatz- und/oder Wirkstoffe, insbesondere Repellentien und/oder Selbstbräuner und/oder Pigmente, enthält.

12. Zubereitung nach einem der vorhergehenden Ansprüche, **dadurch gekennzeichnet, dass** sie eine hohe Wasserfestigkeit aufweist.

13. Verwendung einer Zubereitung nach einem der vorstehenden Ansprüche zum Versprühen auf die Haut.

14. Verwendung einer Zubereitung nach einem der Ansprüche 1 bis 12 als Kosmetikum, insbesondere zur Hautpflege und/oder Insektenabwehr und/oder Selbstbräunung der Haut.

15. Verwendung einer Zubereitung nach einem der Ansprüche 7 bis 12 zur Herstellung eines Sonnenschutzmittels.

16. Verwendung einer Zubereitung nach einem der vorgenannten Ansprüche 1 bis 12 zur Herstellung eines Mittels zum Schutz vor lichtbedingter Hautalterung.

17. Verwendung einer Zubereitung nach einem der vorgenannten Ansprüche 1 bis 12 zur Hautbefeuchtung.

18. Verwendung einer Zubereitung nach mindestens einem der vorgenannten Ansprüche 1 bis 12 zur Anwendung in Pump - Zerstäubern, Handzerstäuberpumpen mit Einfinger- oder "Trigger-Betätigung", Aerosolpumpen, Dispenserpumpen, Quetschflaschen oder in sog. Bag-in-Can Systemen.

## Claims

1. Cosmetic or dermatological preparation based on a low-viscosity W/O emulsion comprising more than 30% by weight of lipids, based on the total mass of the preparation, **characterized in that** the preparation comprises only one emulsifier, Peg-30 dipolyhydroxystearate, and the preparation has a viscosity in the range from 50 to 2000 mPa s, preferably 100 to 600 mPa s.

2. Preparation according to Claim 1, **characterized in that** the lipid fraction is more than 35% by weight, particularly preferably greater than 37.5% by weight and very particularly preferably more than 40% by weight, in each case based on the total weight of the preparation, where emulsifiers, photoprotective filters and other active ingredients and auxiliaries do not count towards the lipid phase of the preparation.

3. Preparation according to Claim 1 or 2,
**characterized in that** the lipids are selected from the group of medium-polar to nonpolar lipids having an interfacial tension towards water of from 20 to 30 mN/m or above 30 mN/m.

4. Preparation according to one of the preceding claims, **characterized in that** dicaprylyl carbonate, butylene glycol dicaprylate/dicaprate and/or C12-15 alkyl benzoate are selected as lipids.

5. Preparation according to one of the preceding claims, **characterized in that** the fraction of polar lipids having an interfacial tension towards water of less than 20 mN/m is less than 25% by weight, preferably less than 15% by weight, based on the total mass of lipids.

6. Preparation according to one of the preceding claims, **characterized in that** the phase ratio between oil phase and water phase is in the range from 30:70 to 60:40, in particular 35:65 to 50:50, particularly preferably 40:60, where emulsifiers, photoprotective filters and other active ingredients or auxiliaries do not count towards the lipid phase of the preparation.

7. Preparation according to one of the preceding claims, **characterized in that** one or more photoprotective filters, in particular UVA and/or UVB filters, are additionally present.

8. Preparation according to Claim 7, **characterized in that** ethylhexyl methoxycinnamate, phenyl-benzimidazolesulphonic acid, ethylhexyltriazone, bis-ethylhexyloxyphenol methoxyphenyltriazine, butylmethoxydibenzoylmethane and/or titanium dioxide are selected as photoprotective filters.

9. Preparation according to Claim 7 or 8,
**characterized in that** the fraction of photoprotective filters is up to 25% by weight, preferably between 17 and 23% by weight, based on the total mass of the preparation.

10. Preparation according to Claim 7, 8 or 9,
**characterized in that**
- 17-23% by weight, preferably 20% by weight, of photoprotective filter,
- 35-45% by weight, preferably 40% by weight, of lipids and
- 2-7% by weight, preferably 5% by weight, of emulsifier are present, in each case based on the total mass of the preparation.

11. Preparation according to one of the preceding claims, **characterized in that** it comprises one or more additional additives and/or active ingredients, in particular repellents and/or self-tanning agents and/or pigments.

12. Preparation according to one of the preceding claims, **characterized in that** it has a high water resistance.

13. Use of a preparation according to one of the preceding claims for spraying onto the skin.

14. Use of a preparation according to one of Claims 1 to 12 as cosmetic, in particular for skincare and/or self-tanning of the skin.

15. Use of a preparation according to one of Claims 7 to 12 for producing a sunscreen composition.

16. Use of a preparation according to one of the preceding Claims 1 to 12 for producing a composition for protection against photoinduced skin ageing.

17. Use of a preparation according to one of the preceding Claims 1 to 12 for moisturizing the skin.

18. Use of a preparation according to at least one of the preceding Claims 1 to 12 for use in atomizers, manual atomizer pumps with single-finger or "trigger operation", aerosol pumps, dispenser pumps, squeezy bottles or in so-called bag-in-can systems.

## Revendications

1. Préparation cosmétique ou dermatologique à base d'une émulsion E/H fluide comprenant plus de 30 % en poids de lipides, par rapport à la masse totale de la préparation, **caractérisée en ce que** la préparation ne comprend qu'un émulsifiant, PEG-30 dipolyhydroxy-stéarate, et la préparation présente une viscosité dans la plage de 50 à 2 000 mPa.s, de préférence de 100 à 600 mPa.s.

2. Préparation selon la revendication 1,
**caractérisée en ce que** la proportion de lipides est de plus de 35 % en poids, de façon particulièrement préférée, supérieure à 37,5 % en poids, et de façon tout particulièrement préférée, de plus de 40 % en poids, chaque fois par rapport au poids total de la préparation, les émulsifiants, les filtres photoprotecteurs et les autres actifs ou adjuvants n'étant pas comptés dans la phase lipidique de la préparation.

3. Préparation selon la revendication 1 ou 2, **caractérisée en ce que** les lipides sont choisis dans le groupe des lipides moyennement à non polaires ayant une tension superficielle vis-à-vis de l'eau de 20 à 30 mN/m ou de plus de 30 mN/m.

4. Préparation selon l'une quelconque des revendications précédentes, **caractérisée en ce que** le carbonate de dicaprylyle, le dicaprylate/dicaprate de butylèneglycol et/ou des benzoates d'alkyle en C₁₂-C₁₅ sont choisis en tant que lipides.

5. Préparation selon l'une quelconque des revendications précédentes, **caractérisée en ce que** la teneur en lipides polaires, ayant une tension superficielle vis-à-vis de l'eau inférieure à 20 mM/m, est inférieure à 25 % en poids, de préférence inférieure à 15 % en poids, par rapport à la masse totale des lipides.

6. Préparation selon l'une quelconque des revendications précédentes, **caractérisée en ce que** le rapport de phases entre phase huileuse et phase aqueuse se situe dans la plage de 30:70 à 60:40, en particulier de 35:65 à 50:50, de façon particulièrement préférée de 40:60, les émulsifiants, les filtres photoprotecteurs et les autres actifs ou adjuvants n'étant pas comptés dans la phase lipidique de la préparation.

7. Préparation selon l'une quelconque des revendications précédentes, **caractérisée en ce qu'**un ou plusieurs filtres photoprotecteurs, en particulier filtres UVA et/ou filtres UVB, sont en outre contenus.

8. Préparation selon la revendication 7,
**caractérisée en ce qu'**on choisit en tant que filtres photoprotecteurs le méthoxycinnamate d'éthylhexyle, l'acide phénylbenzimidazole-sulfonique, l'éthylhexyl-triazone, la bis-éthylhexyloxyphénol méthoxyphényl-triazine, le butylméthoxydibenzoylméthane et/ou le dioxyde de titane.

9. Préparation selon la revendication 7 ou 8, **caractérisée en ce que** la teneur en filtres photoprotecteurs va jusqu'à 25 % en poids, de préférence est comprise entre 17 et 23 % en poids, par rapport à la masse totale de la préparation.

10. Préparation selon la revendication 7, 8 ou 9,
**caractérisée en ce que**
- 17-23 % en poids, de préférence 20 en poids, de filtres photoprotecteurs,
- 35-45 % en poids, de préférence 40 en poids, de lipides et
- 2-7 % en poids, de préférence 5 % en poids, d'émulsifiant sont contenus, chaque fois par rapport à la masse totale de la préparation.

11. Préparation selon l'une quelconque des revendications précédentes, **caractérisée en ce qu'**elle contient un ou plusieurs actifs et/ou additifs supplémentaires, en particulier des insectifuges et/ou des autobronzants et/ou des pigments.

12. Préparation selon l'une quelconque des revendications précédentes, **caractérisée en ce qu'**elle présente une grande résistance à l'eau.

13. Utilisation d'une préparation selon l'une quelconque des revendications précédentes, pour la pulvérisation sur la peau.

14. Utilisation d'une préparation selon l'une quelconque des revendications 1 à 12, en tant que cosmétique, en particulier pour le soin de la peau et/ou la défense contre les insectes et/ou l'autobronzage de la peau.

15. Utilisation d'une préparation selon l'une quelconque des revendications 7 à 12, pour la fabrication d'un produit de protection solaire.

16. Utilisation d'une préparation selon l'une quelconque des revendications 1 à 12, pour la fabrication d'un produit destiné à la protection contre le vieillissement photo-induit de la peau.

17. Utilisation d'une préparation selon l'une quelconque des revendications 1 à 12 précitées, pour l'hydratation de la peau.

18. Utilisation d'une préparation selon au moins l'une des revendications 1 à 12 précitées, pour l'application dans des pulvérisateurs à pompe, des pulvérisateurs à pompe à main à actionnement avec un doigt ou "à gâchette", des pompes aérosol ; des pompes de distributeurs, des flacons à presser ou dans des systèmes dits Bag-in-Can.
